(19) **Europäisches Patentamt**

**European Patent Office**

**Office européen des brevets**

(11) **EP 1 671 636 A1**

(12) **EUROPEAN PATENT APPLICATION**
published in accordance with Art. 158(3) EPC

(43) Date of publication:
**21.06.2006 Bulletin 2006/25**

(51) Int Cl.:
*A61K 31/606* (2000.01)   *A61K 47/02* (1990.01)
*A61K 47/12* (1990.01)   *A61K 47/20* (1990.01)
*A61K 47/22* (1990.01)   *A61P 1/04* (2000.01)

(21) Application number: **04773268.0**

(22) Date of filing: **17.09.2004**

(86) International application number:
**PCT/JP2004/013627**

(87) International publication number:
**WO 2005/027932 (31.03.2005 Gazette 2005/13)**

(84) Designated Contracting States:
**AT BE BG CH CY CZ DE DK EE ES FI FR GB GR
HU IE IT LI LU MC NL PL PT RO SE SI SK TR**

(30) Priority: **22.09.2003 JP 2003329796**

(71) Applicant: **Nisshin Kyorin Pharmaceutical Co. Ltd.
Tokyo 1010054 (JP)**

(72) Inventor: **SHIMIZU, N.,
Nisshin Kyorin Pharma.Co., Ltd.
Fujiminoshi, Saitama 3568511 (JP)**

(74) Representative: **Smyth, Gyles Darren
Marks & Clerk
90 Long Acre
London WC2E 9RA (GB)**

(54) **5-AMINOSALICYLIC ACID SOLID PREPARATION IMPROVED IN DISCOLORATION AND METHOD OF STORING THE SAME**

(57)    It is an object of the present invention to inhibit browning of a 5-aminosalicylic acid solid preparation, and to maintain for an extended period the properties of the 5-aminosalicylic acid solid preparation the same as they were at the time of the manufacturing the preparation. The present invention provides a solid preparation obtained by formulating 5-aminosalicylic acid or a salt thereof and a discoloration inhibitor into a drug product, where- in this solid preparation exhibits a color difference in a CIELAB color space being 10.5 or less before and after storage at 80°C for one week. The above-mentioned discoloration inhibitor contains at least one selected from the group consisting of a thiol compound, a sulfide compound, an acid anhydride, and a hygroscopic compound.

EP 1 671 636 A1

**Description**

Technical Field

This invention relates to a technology for preventing discoloration of a pharmaceutical composition that contains 5-aminosalicylic acid.

Background Art

5-Aminosalicylic acid has been used in the past in the treatment of ulcerative colitis and Crohn's disease, and has been used as a solid preparation having sustained release or gradual release properties in order to reach at the colon or rectum (the diseased site) by oral administration. For instance, there have been proposals for coated granules in which 5-aminosalicylic acid is coated along with an orally administrable carrier with ethyl cellulose or the like (for example, see Patent Document 1: JP-A-S58-501174), and an oral composition in which an anionic polymer such as a carboxyacrylic polymer or the like is used as a coating material (for example, see Patent Document 2: JP-A-S57-500432).

More specifically, Patent Document 1 discloses a solid preparation wherein, in order to put a solid preparation containing 5-aminosalicylic acid into an orally administrable form, 5-aminosalicylic acid (the main ingredient) is mixed with an excipient, a binder, a lubricant, and a disintegrant, and molded into granules or the like, and this product is coated with a film coating agent such as ethyl cellulose and then made into tablets. It is known that light or oxidation can turn these 5-aminosalicylic acid solid preparations brown (hereinafter referred to as "browning"), and measures that have been employed to deal with this browning include light-proof packaging and sealing the preparation along with an oxygen absorber.

Recently, however, it has become apparent that 5-aminosalicylic acid turns brown when stored under high temperatures, such as during the summer months, and that this is unpleasant to the physicians who administer the drug to patients, and to the patients who take the drug.

Recent research into this situation has revealed that 5-aminosalicylic acid is particularly unstable in the presence of an alkali, and is changed by oxidation into 5-aminosalicylic acid quinoneimine, and furthermore that auto-oxidation forms dimers, trimers, quatromers, and higher polymers, causing the material to change to a very deep reddish-brown (for example, see Non-Patent Document 1: J. Jensen et al., International Journal of Pharmaceutics, 88 (1992), 177-187). As a result, it is believed that a 5-aminosalicylic acid solid preparation itself turns brown due to gradual chemical change through oxidation of the preparation as discussed above.

To facilitate storage, shipment, dispensing, and so forth, prior to distribution, a 5-aminosalicylic acid solid preparation is usually packed in a brown bottle along with an oxygen absorber, or is put in strip packaging (so-called "SP package") in which cellophane or low-density polyethylene is used as the packaging material, or is put in a press-through pack (hereinafter referred to as "PTP sheet package").

Also, these preparations have been packaged along with an oxygen absorber in a gas-barrier packaging material such as aluminum foil to prevent browning by light or oxidation. For example, when a 5-aminosalicylic acid solid preparation is stored at a high temperature, the moisture inside the tablets and originating in the additives can evaporate, and that this water vapor accelerates browning, and based on this knowledge, there has been a report in which browning is suppressed by sealing and packaging the preparation, along with a water absorbent and/or a moisture absorbent, in a gas-barrier packaging material, and also using Ageless® (Mitsubishi Gas Chemical) as an oxygen absorber (for example, see Patent Document 3: JP-A-H10-015032).

Nevertheless, improvements to the packaging alone have not been effective at preventing browning after the package has been opened at the pharmacy, so there is a need for some new way to prevent browning.

On the other hand, a stable liquid preparation of 5-aminosalicylic acid has been disclosed (for example, see Patent Document 4: U.S. Patent 4,657,900,; Patent Document 5: JP-A-H3-47161, and Non-Patent Document 2: Lancet, Aug. 8, 1981, 270-271). It has been disclosed that an antioxidant such as ascorbic acid or a sodium salt thereof, a metabisulfite, or EDTA (a metal complexing agent) is effective at preventing the discoloration of the liquid preparation (enema), but it is unclear whether these additives will have the same effect on a 5-aminosalicylic acid solid preparation. In particular, a 5-aminosalicylic acid solid preparation to which a metabisulfite or a hydrogen sulfite salt has been added as an antioxidant contributes to the stabilization of the 5-aminosalicylic acid, but problems such as allergic reactions, a sulfur odor, or the corrosion of packaging may occur depending on the added amount, so these substances are not suited to use as an additive to the solid preparation pertaining to the present invention.

Disclosure of Invention

In light of the above situation, it is an object of the present invention to inhibit the browning of a 5-aminosalicylic acid solid preparation, and to maintain for an extended period the properties of a 5-aminosalicylic acid solid preparation the same as they were at the time of the manufacturing the solid preparation.

As a result of diligent research, the inventor arrived at the present invention upon discovering that the browning of the 5-aminosalicylic acid solid preparation can be suppressed by adding a specific compound to the 5-aminosalicylic acid solid preparation.

More specifically, the present invention provides:

(1) A solid preparation comprising: 5-aminosalicyclic acid or a salt thereof; and a discoloration inhibitor,
(2) The solid preparation according to item (1), wherein a color difference of the solid preparation in a CIELAB color space is 10.5 or less before and after storage at 80°C for one week,
(3) The solid preparation according to item (2), wherein the color difference in the CIELAB color space is 7.0 or less,
(4) The solid preparation according to any one of items (1) to (3), wherein the discoloration inhibitor comprises at least one selected from the group consisting of a thiol compound, a sulfide compound, an acid anhydride, and a hygroscopic compound,
(5) The solid preparation according to item (4), wherein the thiol compound comprises thiomalic acid, thioglycolic acid, L-cysteine, N-acetyl-L-cysteine, or a salt thereof,
(6) The solid preparation according to item (4), wherein the thiol compound comprises L-cysteine or a salt thereof,
(7) The solid preparation according to item (4), wherein the sulfide compound comprises L-cystine, biotin, methionine, or a salt thereof.
(8) The solid preparation according to item (4), wherein the acid anhydride comprises phthalic anhydride, isatoic anhydride, 4,5-dichlorophthalic anhydride, pyromellitic dianhydride, norbornene-2,3-dicarboxylic anhydride, 2,3-pyridinedicarboxylic anhydride, 3,4-pyridinedicarboxylic anhydride, 2,3-naphthalenedicarboxylic anhydride, 5-(2,5-dioxotetrahydrofuryl)-3-cyclohexene-1,2-dicarboxylic anhydride, 1,2,4-benzenetricarboxylic anhydride, diphenic anhydride, or 3,3',4,4'-benzophenonetetracarboxylic dianhydride,
(9) The solid preparation according to item (4), wherein the hygroscopic compound comprises calcium chloride, magnesium chloride, calcium oxide, magnesium oxide, magnesium sulfate, potassium carbonate, calcium carbonate, or anhydrous materials thereof,
(10) The solid preparation according to any of items (1) to (9), wherein the discoloration inhibitor is added in an amount of from 0.1 to 25% by mass based on the 5-aminosalicylic acid or salt thereof,
(11) The solid preparation according to any one items (1) to (10), wherein an average particle size of the discoloration inhibitor is 50 μm or less.

The present invention also provides:

(12) A method for storing a 5-aminosalicylic acid solid preparation, comprising adding a discoloration inhibitor to 5-aminosalicylic acid or a salt thereof,
(13) The method according to item (12), wherein a color difference of the solid preparation in a CIELAB color space is 10.5 or less before and after storage at 80°C for one week,
(14) The method according to item (13), wherein the color difference in the CIELAB color space is 7.0 or less,
(15) The solid preparation according to any one of items (12) to (14), wherein the discoloration inhibitor comprises at least one selected from the group consisting of a thiol compound, a sulfide compound, an acid anhydride, and a hygroscopic compound,
(16) The solid preparation according to item (15), wherein the thiol compound comprises thiomalic acid, thioglycolic acid, L-cysteine, N-acetyl-L-cysteine, or a salt thereof,
(17) The solid preparation according to item (15), wherein the thiol compound comprises L-cysteine or a salt thereof,
(18) The solid preparation according to item (15), wherein the sulfide compound comprises L-cystine, biotin, methionine, or a salt thereof,
(19) The solid preparation according to item (15), wherein the acid anhydride comprises phthalic anhydride, isatoic anhydride, 4,5-dichlorophthalic anhydride, pyromellitic dianhydride, norbornene-2,3-dicarboxylic anhydride, 2,3-pyridinedicarboxylic anhydride, 3,4-pyridinedicarboxylic anhydride, 2,3-naphthalenedicarboxylic anhydride, 5-(2,5-dioxotetrahydrofuryl)-3-cyclohexene-1,2-dicarboxylic anhydride, 1,2,4-benzenetricarboxylic anhydride, diphenic anhydride, or 3,3',4,4'-benzophenonetetracarboxylic dianhydride,
(20) The solid preparation according to item (15), wherein the hygroscopic compound comprises calcium chloride, magnesium chloride, calcium oxide, magnesium oxide, magnesium sulfate, potassium carbonate, calcium carbonate, or anhydrous materials thereof,
(21) The solid preparation according to any of items (12) to (20), wherein the discoloration inhibitor is added in an amount of from 0.1 to 25% by mass based on the 5-aminosalicylic acid or salt thereof,
(22) The solid preparation according to any one of items (12) to (21), wherein an average particle size of the discoloration inhibitor is 50 μm or less.

(23) A method for storing a 5-aminosalicylic acid solid preparation, comprising the steps of: adding L-cysteine to 5-aminosalicylic acid or a salt thereof to produce a 5-aminosalicylic acid solid preparation; and packaging the 5-aminosalicylic acid solid preparation along with an oxygen absorber that exhibits a deoxidization function under an environment of a low humidity.

According to the present invention, a 5-aminosalicylic acid solid preparation can be prevented from browning by adding to the 5-aminosalicylic acid solid preparation a discoloration inhibitor, which particularly has a radical scavenging action, dehydration action, or hygroscopic action.

The term "discoloration inhibitor" as used in the present invention collectively refers to all pharmacologically acceptable compounds that are suited to suppressing the discoloration, and the browning in particular, of 5-aminosalicylic acid or a salt thereof that is an active ingredient of a preparation.

The term "CIELAB color space" as used in the present invention refers to the Lab display system specified by the International Commission on Illumination. With this CIELAB color space (JIS Z 8729), colors are defined by three categories L*, a*, and b*, where L* defines the luminance of a color, while a* and b* both define the hue and saturation characteristics of a given color. The phrase "color difference in the CIELAB color space" (hereinafter referred to as "ΔE*") as used in the present invention defines the difference between two colors before and after the storage of the solid preparation according to the present invention. The greater is ΔE*, the greater is the difference between the two colors.

The term "hygroscopic compound" as used in the present invention refers to a compound having a function of being able to directly absorb and directly retain water vapor or other such moisture, or a compound having a function of being able to absorb and directly retain the water produced by condensation of water vapor or other such moisture.

The term "solid preparation" as used in the present invention encompasses powders, granules, tablets, and other such solid dosage forms.

Advantageous Effects of Invention

According to the solid preparation according to the present invention, browning during storage can be effectively suppressed by adding, for example, at least one compound selected from the group consisting of a thiol compound, a sulfide compound, an acid anhydride, and a hygroscopic compound as a discoloration inhibitor to 5-aminosalicylic acid or a salt thereof.

Brief Description of Drawings

FIG. 1 shows the results of examining the discoloration inhibitory effect in a 5-aminosalicylic acid solid preparation containing the discoloration inhibitor according to the present invention;
FIG. 2 shows the results of examining the discoloration inhibition rate and the color difference of a 5-aminosalicylic acid solid preparation when the discoloration inhibitor according to the present invention was added; and
FIG. 3 shows the results of the color difference of 5-aminosalicylic acid solid preparations when L-cysteine having different average particle sizes was added as the discoloration inhibitor according to the present invention.

Best Mode for Carrying Out the Invention

The following embodiments are examples which are used to describe the present invention, but should not be construed to limit the present invention to just these embodiments. The following embodiments can be modified without departing from the spirit and scope of the present invention.

The solid preparation according to the present invention comprises 5-aminosalicylic acid or a salt thereof, and a discoloration inhibitor. The 5-aminosalicylic acid that is the active ingredient used in the present invention is, for example, commercially available as Pentasa® from Nisshin Kyorin. As will be discussed below, for example, a method for manufacturing 5-aminosalicylic acid sustained release granules is disclosed in WO03/032952.

Salts of 5-aminosalicylic acid used in the present invention include all pharmacologically acceptable acidic salts, examples of which include salts of inorganic acids, such as hydrochloride, hydrobromide, sulfate, nitrate, phosphate, and the like; salts of carboxylic acids, such as acetate, fumarate, maleate, oxalate, malonate, succinate, citrate, malate, and the like; salts of sulfonic acids, such as methanesulfonate, ethanesulfonate, benzenesulfonate, toluenesulfonate, and the like; and salts of amino acids, such as glutamate, aspartate, and the like. Hydrochloride and sulfate are preferable as acidic salts of 5-aminosalicylic acid, and hydrochloride is particularly favorable.

Salts of 5-aminosalicylic acid used in the present invention further include all pharmacologically acceptable basic salts, examples of which include salts of alkali metals, such as lithium salt, sodium salt, potassium salt, and the like; salts of earth metals, such as calcium salt, magnesium salt, and the like; ammonium salt; and salts of organic salts of triethylamine, diisopropylamine, cyclohexylamine, and the like. Salts of alkali metal and ammonium salt are preferable as basic salts of 5-aminosalicylic acid, and sodium salt and ammonium salt are particularly favorable.

Examples of the pharmacologically acceptable discoloration inhibitor used in the present invention include a thiol compound, a sulfide compound, an acid anhydride, and a hygroscopic compound.

Browning during the storage of the solid preparation according to the present invention can be especially well suppressed by adding the discoloration inhibitor used in the present invention. The specific index of browning suppression in the present invention is that suppression of browning is deemed good if the color difference $\Delta E^*$ in the CIELAB color space (JIS Z 8729) is no more than 10.5 before and after the storage of the solid preparation. Preferably, the color difference $\Delta E^*$ is 7.0 or less, and even more preferably the color difference $\Delta E^*$ is 6.5 or less.

Specific examples of the thiol compound include thiomalic acid, thioglycolic acid, L-cysteine, N-acetyl-L-cysteine, glutathione, salts of the foregoing and the like. Thioglycolic acid and sodium thioglycolate, and thiomalic acid, L-cysteine, N-acetyl-L-cysteine, as well as sodium salts, potassium salts, and hydrochlorides of the foregoing, are preferable as the thiol compound.

Specific examples of the sulfide compound include L-cystine, biotin, methionine, and salts of the foregoing.

Specific examples of the acid anhydride include maleic anhydride, succinic anhydride, phthalic anhydride, isatoic anhydride, 4,5-dichlorophthalic anhydride, pyromellitic dianhydride, norbornene-2,3-dicarboxylic anhydride, 2,3-pyridinedicarboxylic anhydride, 3,4-pyridinedicarboxylic anhydride, 2,3-naphthalenedicarboxylic anhydride, 5-(2,5-dioxotetrahydrofuryl)-3-cyclohexene-1,2-dicarboxylic anhydride, 1,2,4-benzenetricarboxylic anhydride, diphenic anhydride, and 3,3',4,4'-benzophenonetetracarboxylic dianhydride. Phthalic anhydride, 3,4-pyridinedicarboxylic anhydride, 2,3-naphthalenedicarboxylic anhydride, and 3,3',4,4'-benzophenonetetracarboxylic dianhydride are preferable as the acid anhydride.

Specific examples of the hygroscopic compound include calcium chloride, magnesium chloride, calcium oxide, magnesium oxide, magnesium sulfate, potassium carbonate, calcium carbonate, and anhydrous materials of the foregoing, hydrates of the foregoing, and the like. Anhydrous magnesium chloride is preferable as the hygroscopic compound.

Various compounds, either singly or in combinations, can be used as the discoloration inhibitor according to the present invention.

An amount in which the discoloration inhibitor used in the present invention is added is generally from 0.1 to 100 % by mass, and preferably from 0.1 to 25 % by mass, and even more preferably from 0.1 to 1 % by mass, based on the 5-aminosalicylic acid or salt thereof.

The discoloration inhibitor used in the present invention preferably has a more pronounced discoloration inhibitory effect when its average particle size is smaller. The average particle size is preferably 50 $\mu$m or less, and more preferably 40 $\mu$m or less, with 30 $\mu$m or less being still more preferably.

There are no particular restrictions on the mode of administration of the solid preparation according to the present invention, but oral administration is preferable. Favorable dosage forms that can be used for oral administration include tablets, coated tablets, pills, fine granules, coarse granules, powders, and capsules.

There are no particular restrictions on the packaging form of the solid preparation according to the present invention, but it is preferable if a preparation containing a discoloration inhibitor is packaged in an airtight container, or packaged in an ordinary PTP package (PVC/AL), or packaged in an aluminum packet.

Specific examples of airtight containers that can be used in the present invention include containers that are substantially impervious to gases and whose seal can be broken, such as an aluminum container, or a bottle that blocks light, such as a brown bottle, and there are no limitations on the shape or material of the container.

The material used for the packaging can be a breathable packaging material (such as cellophane, low-density polyethylene film (density: 0.91 to 0.93 g/mL), polyvinyl chloride film, polypropylene film, paper, low-density polyethylene-laminated paper, polypropylene-laminated paper, synthetic paper, and the like), or a material with gas barrier properties (such as aluminum foil, high-density polyethylene film (density: 0.95 to 0.97 g/mL), polyvinylidene chloride film, high-density polyethylene-laminated paper, or polyvinylidene chloride-laminated paper), but the present invention is not limited to these. A preparation containing one of the above-mentioned thiol compounds can be prevented from giving off a sulfur odor by using a packaging material with gas barrier properties.

It is also favorable for the packaging to include a deodorant, desiccant, and oxygen absorber. In particular, including a deodorant, desiccant, and oxygen absorber in the packaging is effective at eliminating the sulfur odor of a preparation containing one of the above-mentioned thiol compounds.

Examples of deodorants that can be used include, but are not limited to, synthetic zeolite and activated carbon.

Desiccants include water absorbents and moisture absorbents. Highly water-absorbent resins and the like are favorable as water absorbents, examples of which include, but are not limited to, saponified copolymers of a vinyl ester and an ethylenic unsaturated carboxylic acid or derivative thereof (more specifically, a saponified copolymer of vinyl acetate and a (meth)acrylic ester, a saponified copolymer of vinyl acetate and maleic anhydride, or the like); graft polymers obtained by grafting (meth)acrylic acid, maleic acid, crotonic acid, or other such unsaturated carboxylic acid or a derivative thereof to a polysaccharide such as starch or cellulose; mixtures of one of the above-mentioned graft polymers and insolubilized carboxymethyl cellulose; a saponified copolymer of isobutylene and maleic anhydride; a copolymer of acrylic acid and methacrylic acid; and a three-dimensional moisture absorbent obtained by crosslinking a hydrophilic polymer such as polyvinyl alcohol, polyethylene oxide, polypropylene oxide, polyvinyl pyrrolidone, sulfonated polystyrene, polyvinyl pyridine, polyacrylamide, polymethacrylamide, or the like.

Examples of moisture absorbents include, but are not limited to, calcium chloride, calcium carbonate, silica gel, magnesium carbonate, magnesium aluminate silicate, and other neutral moisture absorbents, calcium oxide, calcium hydroxide, and other basic moisture absorbents, and activated carbon, porous zeolite, and other porous moisture absorbents. Calcium chloride and silica gel can be used preferably as a moisture absorbent. Alternatively, according to need, two or more different moisture absorbents can be used at the same time.

Examples of oxygen absorbers include iron powder-based oxygen absorbers such as Ageless® (Mitsubishi Gas Chemical), and oxygen absorbers that exhibit a deoxidization function under an environment of a low humidity (such as the activated transition metal (such as manganese, iron, cobalt, or copper) disclosed in JP-A-H8-38883, the reductive metal and metal iodide, or reductive metal and metal bromide, disclosed in JP-A-H10-309427, the low-molecular weight phenol compound and activated carbon disclosed in JP-A-2000-50849, the iron powder / iodine or iron powder / iodine / metal iodide disclosed in JP-A-2000-50850, the activated magnesium disclosed in JP-A-2001-37457, or the organic readily-oxidizable composition and silicon dioxide disclosed in JP-A-2003-38143). It is especially favorable for the oxygen absorber used in the present invention to be a reductive metal and a metal iodide, or a reductive metal and a metal bromide, or iron powder /iodine or iron powder / iodine / metal iodide. Best of all is to use commercially available PharmaKeep® (Mitsubishi Gas Chemical).

The dosage of the solid preparation according to the present invention can be suitably determined in accordance with the symptoms, age, weight, and other such conditions, but the adult dose is generally from 500 to 5000 mg/day, and preferably from 1000 to 4500 mg/day, and more preferably from 1500 to 4000 mg/day.

The solid preparation according to the present invention can be obtained by an conventional formulation method, and can contain commonly used excipients (such as crystalline cellulose, lactose, sucrose, starch, mannitol or the like), binders (such as gum arabic, carboxymethyl cellulose, polyvinyl pyrrolidone or the like), disintegrants (such as calcium carbonate, carboxymethyl cellulose calcium or the like), lubricants (such as magnesium stearate, talc or the like), flavorings (such as ordinarily used sweeteners, acidifiers, spices or the like), and other such additives. This solid preparation can be formulated by a standard method involving the mixing of components commonly used as raw materials for pharmaceutical preparations.

More specifically, a pharmacologically acceptable discoloration inhibitor and other additives are added to 5-aminosalicylic acid or a salt thereof, and the components are granulated in the presence of a solvent to obtain granules or a powder. Thereafter, this product can be molded into tablets.

Tablets can also be molded by adding a discoloration inhibitor and other additives to a granulated material obtained by coating granules of 5-aminosalicylic acid with ethyl cellulose or the like as disclosed in WO 81/02671 and WO 03/32952.

Next, the storage method according to the present invention will be described. A 5-aminosalicylic acid solid preparation containing the discoloration inhibitor of the present invention and 5-aminosalicylic acid or a salt thereof undergoes less browning of the 5-aminosalicylic acid during high-temperature storage than with a 5-aminosalicylic acid solid preparation that does not contain the above-mentioned discoloration inhibitor.

Reference examples, working examples, and comparative examples will now be given to illustrate the beneficial effects of the discoloration inhibitor according to the present invention, but these are given for illustrative purposes, and the present invention is in no case limited to the following specific examples. All percentages are by mass unless otherwise specified.

Preparation Example: Preparation of sustained-release granules

A 10% aqueous solution of povidone was added to and kneaded with 1000 g of 5-aminosalicylic acid, and this mixture was granulated by extrusion granulation and then dried. The dried granules were sieved to obtain crude granules of 10 to 30 mesh. Next, 1000 g of a 1% aqueous solution ethyl cellulose was sprayed in a fluidized layer onto 500 g of crude granules, and followed by drying and sifting to obtain sustained-release granules of 10 to 30 mesh. The sustained-release granules thus obtained had a composition of 94.0% 5-aminosalicylic acid, 5.0% povidone, and 1.0% ethyl cellulose.

Reference Example 1

Preparation of Mixed Powder

9.85 g of crystalline cellulose and 0.15 g of magnesium stearate were added to 20 g of the sustained-release granules obtained in the above preparation example, and these components were mixed to obtain a mixture.

Storage Test

5 g of the above mixture was put in a glass bottle and the bottle was capped. The bottle was then stored for one week at 80°C. The coloring (L*, a*, b*) of the mixture before and after storage was measured with a spectrophotometer (Spectrophotometer CM-3500d; Minolta Co. Ltd.), and the color difference ($\Delta E^*$) before and after storage was calculated from the following formula:

$$\Delta E^* = \sqrt{(L_1^* - L_2^*)^2 + (a_1^* - a_2^*)^2 + (b_1^* - b_2^*)^2}$$

formula (1)

wherein $L_1^*$, $a_1^*$, and $b_1^*$ are the color values before storage, while $L_2^*$, $a_2^*$, and $b_2^*$ are the color values after storage.

Working Example 1

Preparation of Mixed Powder

6.85 g of crystalline cellulose, 0.15 g of magnesium stearate, and 3 g of discoloration inhibitor were added to 20 g of the sustained-release granules obtained in the above preparation example, and these components were mixed to obtain a mixture.

Storage Test

5 g of the above mixture was put in a glass bottle and the bottle was capped, then the mixture was stored at 80°C for one week. The coloring before and after storage was measured and the color difference before and after storage was calculated in the same manner as in Reference Example 1.
The discoloration inhibition rate was calculated to be 100% when the color difference was 0, and 0% when the color difference was over the value in Reference Example 1.

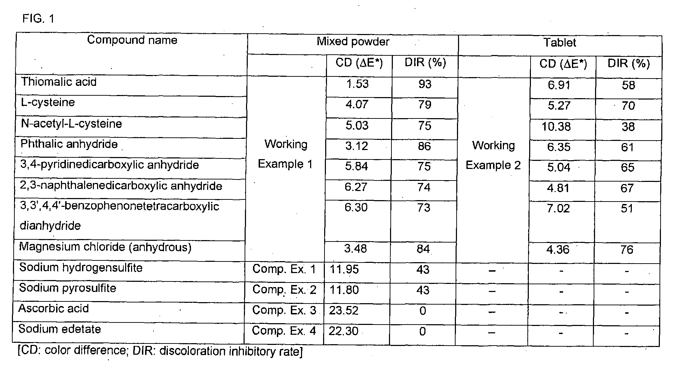

FIG. 1 shows the results for discoloration inhibition rate and color difference of 5-aminosalicylic acid solid preparations when the discoloration inhibitor according to the present invention were added.

Comparative Example 1

A mixture was prepared and a storage test conducted in the same manner as in Working Example 1, except that sodium hydrogensulfite was used instead of the discoloration inhibitor according to the present invention. These results are given in FIG. 1.

Comparative Example 2

A mixture was prepared and a storage test conducted in the same manner as in Working Example 1, except that sodium pyrosulfite was used instead of the discoloration inhibitor according to the present invention. These results are given in FIG. 1.

Comparative Example 3

A mixture was prepared and a storage test conducted in the same manner as in Working Example 1, except that ascorbic acid was used instead of the discoloration inhibitor according to the present invention. These results are given in FIG. 1.

Comparative Example 4

A mixture was prepared and a storage test conducted in the same manner as in Working Example 1, except that sodium edetate was used instead of the discoloration inhibitor according to the present invention. These results are given in FIG. 1.

Reference Example 2

Preparation of Tablets

300 g of the powder obtained in Reference Example 1 was molded into tablets having a diameter of 9 mm with a compressor (Riken Hydraulic Power P-1 B; Riken Seiki Co. Ltd.).

Storage Test

Five of the tablets obtained in the preparation of the above tablets were put in a glass bottle and the bottle was capped, after which the bottle was stored for one week at 80°C.
The coloring (L*, a*, b*) before and after storage was measured with a spectrophotometer (Spectrophotometer CM-3500d; Minolta Co. Ltd.), and the color difference ($\Delta E^*$) before and after storage was calculated from the above formula 1.

Working Example 2

Preparation of Tablets

300 g of the powder obtained in Working Example 1 was molded into tablets having a diameter of 9 mm with a compressor (Riken Hydraulic Power P-1 B; Riken Seiki Co. Ltd.).

Storage Test

Five of the tablets obtained in the preparation of the above tablets were put in a glass bottle and the bottle was capped, after which the bottle was stored for one week at 80°C.
The coloring (L*, a*, b*) before and after storage was measured with a spectrophotometer (Spectrophotometer CM-3500d; Minolta Co. Ltd.), and the color difference ($\Delta E^*$) before and after storage was calculated from the above formula 1. Note that the color difference value was the average for the above-mentioned five tablets.
The discoloration inhibition rate was calculated to be 100% when the color difference was 0, and 0% when the color difference was over the value in Reference Example 2.
FIG. 1 shows the results for discoloration inhibition rate and color difference ($\Delta E^*$) obtained in Working Example 2.
It is clear from the results in FIG. 1 that the 5-aminosalicylic acid solid preparations containing the discoloration inhibitor according to the present invention had a color difference ($\Delta E^*$) before and after one week of storage at 80°C of 10.5 or less, and that this color difference was less than that in Comparative Examples 1 to 4. The reason for this is surmised to be that the browning of the 5-aminosalicylic acid that is the active ingredient of the solid preparation was suppressed by the discoloration inhibitor according to the present invention. The suppression of browning in the 5-aminosalicylic acid by the discoloration inhibitor according to the present invention was particularly pronounced with a mixed powder.

Reference Example 3

Preparation of Tablets

Crystalline cellulose, a lubricant, etc., were added to and mixed with approximately 21 g of the sustained-release granules obtained in the preparation example above, which gave 30 g of mixed powder. 375 mg of mixed powder was molded into tablets having a diameter of 9 mm with a compressor (Riken Hydraulic Power P-1 B; Riken Seiki Co. Ltd.).

Storage Test

Ten of the tablets obtained in the preparation of the above tablets were put in a glass bottle and the bottle was capped, after which the bottle was stored for one week at 80°C.

The coloring (L*, a*, b*) before and after storage was measured with a spectrophotometer (Spectrophotometer CM-3500d; Minolta Co. Ltd.), and the color difference (ΔE*) before and after storage was calculated from the above formula 1. Note that the color difference value was the average for the above-mentioned ten tablets.

Working Example 3

Preparation of Tablets

0.16 g of discoloration inhibitor, and crystalline cellulose, a lubricant, etc., were added to and mixed with approximately 21 g of the sustained-release granules obtained in the preparation example above, which gave 30 g of mixed powder. 375 mg of mixed powder was molded into tablets having a diameter of 9 mm with a compressor (Riken Hydraulic Power P-1 B; Riken Seiki Co. Ltd.).

Storage Test

Ten of the tablets obtained in the preparation of the above tablets were put in a glass bottle and the bottle was capped, after which the bottle was stored for one week at 80°C.

The coloring (L*, a*, b*) before and after storage was measured with a spectrophotometer (Spectrophotometer CM-3500d; Minolta Co. Ltd.), and the color difference (ΔE*) before and after storage was calculated from the above formula 1. Note that the color difference value was the average for the above-mentioned ten tablets.

The discoloration inhibition rate was calculated to be 100% when the color difference was 0, and 0% when the color.difference was over the value in Reference Example 3.

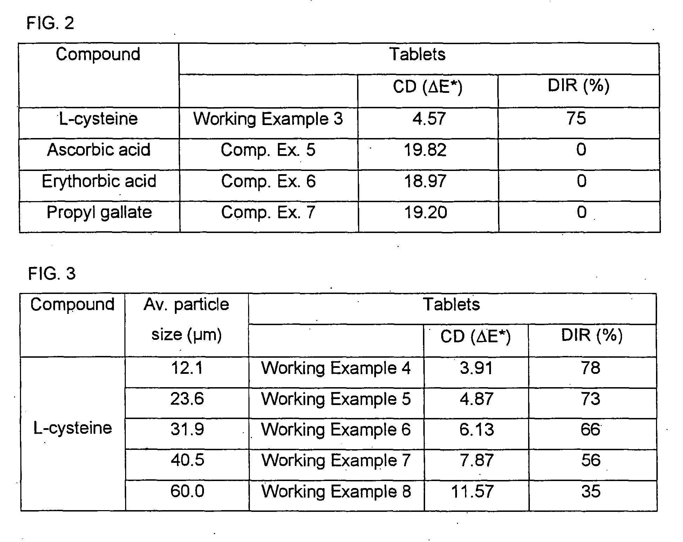

FIG. 2 shows the results for discoloration inhibition rate and color difference of 5-aminosalicylic acid solid preparations when the discoloration inhibitor according to the present invention was added.

Comparative Example 5

Tablets were prepared and a storage test conducted in the same manner as in Working Example 3, except that ascorbic acid was used instead of the discoloration inhibitor according to the present invention. These results are given in FIG. 2.

Comparative Example 6

Tablets were prepared and a storage test conducted in the same manner as in Working Example 3, except that erythorbic acid was used instead of the discoloration inhibitor according to the present invention. These results are given in FIG. 2.

Comparative Example 7

Tablets were prepared and a storage test conducted in the same manner as in Working Example 3, except that propyl gallate was used instead of the discoloration inhibitor according to the present invention. These results are given in FIG. 2.

It is clear from the results in FIG. 2 that the 5-aminosalicylic acid solid preparations containing the discoloration inhibitor according to the present invention had a color difference (ΔE*) before and after one week of storage at 80°C of 5.0 or less, and that this color difference was less than that in Comparative Examples 5 to 7. This indicates that even if the discoloration inhibitor according to the present invention is added in a small amount of approximately 0.8% based on the 5-aminosalicylic acid, a discoloration inhibitory effect will still be observed in the 5-aminosalicylic acid solid preparation.

Working Example 4

Preparation of Tablets

8 g of a discoloration inhibitor with an average particle size of 12.1 μm, and crystalline cellulose, a lubricant, etc., were added to and mixed with 2128 g of the sustained-release granules obtained in the preparation example above, which gave 3000 g of mixed powder. This was molded into tablets with a diameter of 9.5 mm with a rotary tableting machine (Clean Press Correct; Kikusui Seisakusho Co. Ltd.).

Storage Test

Ten of the tablets obtained in the preparation of the above tablets were put in a glass bottle and the bottle was capped, after which the bottle was stored for one week at 80°C.

The coloring (L*, a*, b*) of the tablets before and after storage was measured with a spectrophotometer (Spectrophotometer CM-3500d; Minolta Co. Ltd.), and the color difference ($\Delta E^*$) before and after storage was calculated from the above formula 1. Note that the color difference value was the average for the above-mentioned ten tablets.

FIG. 3 shows the results for color difference of 5-aminosalicylic acid solid preparations when the discoloration inhibitor according to the present invention was added.

Working Example 5

Tablets were prepared and a storage test was conducted in the same manner as in Working Example 4, except that a discoloration inhibitor with an average particle size of 23.6 $\mu$m was used instead of the discoloration inhibitor with the average particle size of 12.1 $\mu$m. These results are given in FIG. 3.

Working Example 6

Tablets were prepared and a storage test was conducted in the same manner as in Working Example 4, except that a discoloration inhibitor with an average particle size of 31.9 $\mu$m was used instead of the discoloration inhibitor with the average particle size of 12.1 $\mu$m. These results are given in FIG. 3.

Working Example 7

Tablets were prepared and a storage test was conducted in the same manner as in Working Example 4, except that a discoloration inhibitor with an average particle size of 40.5 $\mu$m was used instead of the discoloration inhibitor with the average particle size of 12.1 $\mu$m. These results are given in FIG. 3.

Working Example 8

Tablets were prepared and a storage test was conducted in the same manner as in Working Example 4, except that a discoloration inhibitor with an average particle size of 60.0 $\mu$m was used instead of the discoloration inhibitor with the average particle size of 12.1 $\mu$m. These results are given in FIG. 3.

It is clear from the results in FIG. 3 that the discoloration inhibitory effect of the discoloration inhibitor according to the present invention increased in inverse proportion to the particle size, and in particular, the 5-aminosalicylic acid solid preparation containing the discoloration inhibitor with the average particle size of 50 $\mu$m or less had a color difference ($\Delta E^*$) before and after one week of storage at 80°C of 10.5 or less. Also, in particular, the 5-aminosalicylic acid solid preparation containing the discoloration inhibitor with the average particle size of 40 $\mu$m or less had a color difference ($\Delta E^*$) before and after one week of storage at 80°C of 7.0 or less.

Industrial Applicability

According to the present invention, the 5-aminosalicylic acid solid preparation containing the discoloration inhibitor can be stored for an extended period while its properties are kept the same as they were at the time of manufacturing the preparation, which reduces unpleasantness felt by the physicians who administer the drug to patients, and by the patients who take the drug.

**Claims**

1. A solid preparation comprising:

   5-aminosalicyclic acid or a salt thereof; and
   a discoloration inhibitor.

2. The solid preparation according to Claim 1, wherein a color difference of the solid preparation in a CIELAB color space is 10.5 or less before and after storage at 80°C for one week.

3. The solid preparation according to Claim 2, wherein the color difference in the CIELAB color space is 7.0 or less.

4. The solid preparation according to any one of Claims 1 to 3, wherein the discoloration inhibitor comprises at least one selected from the group consisting of a thiol compound, a sulfide compound, an acid anhydride, and a hygroscopic compound.

5. The solid preparation according to Claim 4, wherein the thiol compound comprises thiomalic acid, thioglycolic acid, L-cysteine, N-acetyl-L-cysteine, or a salt thereof.

6. The solid preparation according to Claim 4, wherein the thiol compound comprises L-cysteine or a salt thereof.

7. The solid preparation according to Claim 4, wherein the sulfide compound comprises L-cystine, biotin, methionine, or a salt thereof.

8. The solid preparation according to Claim 4, wherein the acid anhydride comprises phthalic anhydride, isatoic anhydride, 4,5-dichlorophthalic anhydride, pyromellitic dianhydride, norbornene-2,3-dicarboxylic anhydride, 2,3-pyridinedicarboxylic anhydride, 3,4-pyridinedicarboxylic anhydride, 2,3-naphthalenedicarboxylic anhydride, 5-(2,5-dioxotetrahydrofuryl)-3-cyclohexene-1,2-dicarboxylic anhydride, 1,2,4-benzenetricarboxylic anhydride, diphenic anhydride, or 3,3',4,4'-benzophenonetetracarboxylic dianhydride.

9. The solid preparation according to Claim 4, wherein the hygroscopic compound comprises calcium chloride, magnesium chloride, calcium oxide, magnesium oxide, magnesium sulfate, potassium carbonate, calcium carbonate, or anhydrous materials thereof.

10. The solid preparation according to any one of Claims 1 to 3, wherein the discoloration inhibitor is added in an amount of from 0.1 to 25% by mass based on the 5-aminosalicylic acid or salt thereof.

11. The solid preparation according to Claim 10, wherein an average particle size of the discoloration inhibitor is 50 $\mu$m or less.

12. A method for storing a 5-aminosalicylic acid solid preparation, comprising adding a discoloration inhibitor to 5-aminosalicylic acid or a salt thereof.

13. A method for storing a 5-aminosalicylic acid solid preparation, comprising the steps of:

adding L-cysteine to 5-aminosalicylic acid or a salt thereof to produce a 5-aminosalicylic acid solid preparation; and

packaging the 5-aminosalicylic acid solid preparation along with an oxygen absorber that exhibits a deoxidization function under an environment of a low humidity.

FIG. 1

| Compound name | Mixed powder | | | Tablet | | |
|---|---|---|---|---|---|---|
| | | CD (ΔE*) | DIR (%) | | CD (ΔE*) | DIR (%) |
| Thiomalic acid | | 1.53 | 93 | | 6.91 | 58 |
| L-cysteine | | 4.07 | 79 | | 5.27 | 70 |
| N-acetyl-L-cysteine | | 5.03 | 75 | | 10.38 | 38 |
| Phthalic anhydride | Working Example 1 | 3.12 | 86 | Working Example 2 | 6.35 | 61 |
| 3,4-pyridinedicarboxylic anhydride | | 5.84 | 75 | | 5.04 | 65 |
| 2,3-naphthalenedicarboxylic anhydride | | 6.27 | 74 | | 4.81 | 67 |
| 3,3',4,4'-benzophenonetetracarboxylic dianhydride | | 6.30 | 73 | | 7.02 | 51 |
| Magnesium chloride (anhydrous) | | 3.48 | 84 | | 4.36 | 76 |
| Sodium hydrogensulfite | Comp. Ex. 1 | 11.95 | 43 | — | - | - |
| Sodium pyrosulfite | Comp. Ex. 2 | 11.80 | 43 | — | - | - |
| Ascorbic acid | Comp. Ex. 3 | 23.52 | 0 | — | - | - |
| Sodium edetate | Comp. Ex. 4 | 22.30 | 0 | — | - | - |

[CD: color difference; DIR: discoloration inhibitory rate]

FIG. 2

| Compound | Tablets | | |
|---|---|---|---|
| | | CD (ΔE*) | DIR (%) |
| L-cysteine | Working Example 3 | 4.57 | 75 |
| Ascorbic acid | Comp. Ex. 5 | 19.82 | 0 |
| Erythorbic acid | Comp. Ex. 6 | 18.97 | 0 |
| Propyl gallate | Comp. Ex. 7 | 19.20 | 0 |

FIG. 3

| Compound | Av. particle size (μm) | Tablets | | |
|---|---|---|---|---|
| | | | CD (ΔE*) | DIR (%) |
| L-cysteine | 12.1 | Working Example 4 | 3.91 | 78 |
| | 23.6 | Working Example 5 | 4.87 | 73 |
| | 31.9 | Working Example 6 | 6.13 | 66 |
| | 40.5 | Working Example 7 | 7.87 | 56 |
| | 60.0 | Working Example 8 | 11.57 | 35 |

**INTERNATIONAL SEARCH REPORT**

International application No.

PCT/JP2004/013627

A.  CLASSIFICATION OF SUBJECT MATTER
Int.Cl$^7$ A61K31/606, 47/02, 47/12, 47/20, 47/22, A61P1/04

According to International Patent Classification (IPC) or to both national classification and IPC

B.  FIELDS SEARCHED

Minimum documentation searched (classification system followed by classification symbols)
Int.Cl$^7$ A61K31/606, 47/02, 47/12, 47/20, 47/22, A61P1/04

Documentation searched other than minimum documentation to the extent that such documents are included in the fields searched

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| Y | Joan Jensen, Identification of major degradation products of 5-aminosalicylic acid formed in aqueous solutions and in pharmaceuticals, International Journal of Pharmaceutics, 1992, Vol.88, pages 177 to 187 | 1-13 |
| Y | JP 10-15032 A (Nisshin Flour Milling Co., Ltd.), 20 January, 1998 (20.01.98), Claims 1 to 5; Par. Nos. [0003], [0014] (Family: none) | 1-4,9-13 |
| Y | US 2442461 A (Walter Karrer), 01 June, 1948 (01.06.48), Claim 1; column 1, lines 16 to 32 (Family: none) | 1-6,10-13 |

☒ Further documents are listed in the continuation of Box C.       ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 14 October, 2004 (14.10.04) | 02 November, 2004 (02.11.04) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

| International application No. |
|---|
| PCT/JP2004/013627 |

C (Continuation). DOCUMENTS CONSIDERED TO BE RELEVANT

| Category* | Citation of document, with indication, where appropriate, of the relevant passages | Relevant to claim No. |
|---|---|---|
| Y | WO 01/85147 A2 (ELI LILLY AND CO.), 15 November, 2001 (15.11.01), Claims 1 to 3 & JP 2003-535827 A & EP 1357903 A2 | 1-7,10-13 |
| Y | JP 8-81360 A (Wakamoto Pharmaceutical Co., Ltd.), 26 March, 1996 (26.03.96), Claim 1; Par. Nos. [0004], [0006] & EP 700678 A1 & US 5693337 A | 1-4,7,10-13 |
| Y | JP 3-47121 A (Takeda Chemical Industries, Ltd.), 28 February, 1991 (28.02.91), Page 2, upper right column, line 2 to lower right column, line 16 & EP 393537 A2 & US 5077310 A | 1-4,8,10-13 |

Form PCT/ISA/210 (continuation of second sheet) (January 2004)